Europäisches Patentamt

European Patent Office

Office européen des brevets

(11) Veröffentlichungsnummer: **0 582 026 A1**

## EUROPÄISCHE PATENTANMELDUNG

(21) Anmeldenummer: **92830438.5**

(22) Anmeldetag: **06.08.92**

(51) Int. Cl.5: **A61K 9/00**, A61F 6/04, A61K 47/00

(43) Veröffentlichungstag der Anmeldung:
**09.02.94 Patentblatt 94/06**

(84) Benannte Vertragsstaaten:
**AT BE CH DE DK ES FR GB GR IT LI LU MC NL PT SE**

(71) Anmelder: **Ansaloni, Paolo**
**Via Fondazza 23**
**I-40125 Bologna(IT)**
Anmelder: **Cervelli, Simonetta**
**Via Fondazza 23**
**I-40125 Bologna(IT)**

(72) Erfinder: **Ansaloni, Paolo**
**Via Fondazza 23**
**I-40125 Bologna(IT)**
Erfinder: **Cervelli, Simonetta**
**Via Fondazza 23**
**I-40125 Bologna(IT)**

(74) Vertreter: **Rinaldi, Carlo**
**c.o. Studio Brevetti Nazionali ed Esteri**
**dell'Ing. Carlo Rinaldi & C. s.d.f.**
**Piazza di Porta Castiglione, 16**
**I-40136 Bologna (IT)**

(54) **Gleitmittel für Präservative.**

(57) Ein Gleitmittel für Präservative aus einem oder mehreren fetten oder ölhatigen Pflanzenstoffen besteht; einer der Karitene enthaltenden Pflanzenstoffe ist Karitébutter; in wesentlichem die Pflanzenstoffe, die das Gleitmittel zusammensetzen, enthalten Glizeride und Fettsäuren und wenigstens ein Bindemittel zur Besserung der Haftfestigkeit des Gleitmittels an den Außen und Innnenwänden des Präservativs, wobei die Bindemittel die therapeutischen oder lindernden Eigenschaften des Gleitmittels nicht verändern; vorzugsweise sorgt das Lezithin für Bindemittel, das als Emulgiermittel wirkt; vorteilhafterweise einer der Bestandteile (z. B. das Yoyobaöl) Vitamine enthält; vorteilhafterweise besteht das Gleitmittel aus einer Mischung, die wenigstens ein der folgenden Fette oder Öle enthält: Kakaobutter, Borneumtalg, Karitébutter, Mowrafett, Pflanzentalg des China, Muskatnuß-butter.

Die vorliegende Erfindung betrifft ein Gleitmittel für Präservative, das in wesentlichem aus einem Pflanzenfett oder aus einer Mischung von Pflanzenfetten und Pflanzenölen besteht, wobei die Mischung Bindemittel zur Besserung der Haftfestigkeit des Gleitmittels an den Außen-und Innnenwänden des Präservativs enthält.

Aus dem übrigen z. Z. Stand der Technik sind Präservative bekannt, die mit fetten oder öligen Schmierungsstoffen geschmiert sind; diese mineralischen Schmierungsstoffe bestehen in wesentlichem aus Paraffinöl oder aus Vaseline und sorgen für die Erleichterung des Beischlafs und für die Behinderung der Schädigungen der Scheidenchleimhaut

Der Hauptnachteil der bekannten Gleitmittel für Präservative ist in wesentlichem darin zu sehen, daß sie die in der Scheidenchleimhaut vorher bestehnden Bakterien nicht bekämpfen, sondern sie sich beschränken, deren Wurzelfassen zu verhindern.

Hier will die Erfindung Abhilfe schaffen.

Die Erfindung, wie sie in dem unabhängigen Anspruch gekennzeichnet ist, löst die Aufgabe, ein Gleitmittel für Präservative zu schaffen, durch das folgende Vorteile erreicht werden: die vorher bestehnden eventuellen Entzündungen der Scheidenchleimhaut gelindert sind; es schützt die zarten Teile der Scheidenchleimhaut vor der Berührung mit dem Latex des Präservativs, damit läßt sich ein heilsames Ergebnis für den weiblichen Geschlechtsapparat erreichen.

Die durch die Verwendung dieses Gleitmittels erreichten Vorteile sind in wesentlichem darin zu sehen, daß es für die Vernarbung, die Hydration und die Besserung der Elastizität des Hepithels sorgt, da es die Zeilenernährung reaktiviert. Außerdem kuriert es Geschwüre, Ekzeme und Schrunden.

Das erfindungsgemäße Gleitmittel besteht aus einem in wesentlichem aus Triglizeriden gebildet Pflanzenfett, durch dessen Hydrolise sich mehrere Fettsäuren erreichen; diese Fettsäuren sind die Palmitinsäure, die Stearinsäure, die Oleinsäure, die Linoleinsäure und so weiter. Der kennzeichnende Teil dieses Pflanzenfettes besteht aus einer prozentual bedeutenden Fraktion, die unverseifbare genannte Fraktion; der Gehalt an unverseifbaren Stoffen in dem bezüglichen Pflanzenfett ist zwischen 2,5 und 15 Prozent begrenzt; seinerseits enthält dieser Gehalt 1 Prozent an Tritorpenischealkoholen (Lupeol, Basseol, Butyrospermol, Amirine und Perkaol) 0,5 Prozent an Karitene (ein fester Kohlenwasserstoff $C_{32} H_{56}$), die Provitamineigeschaften aufweist, und 0,1 Prozent an Fitosterolen.

Eine derartige chemische Zusammensetzung ist in der Karitébutter (oder Sheabutter) enthalten. Die Anwendung dieses Stoffes als Gleitmittel für Präservative folgt aus der Erfolg der Kapseln, die innerrlich der Scheiden anzuwenden sind; diese Gelatinekapseln sind mit reiner Karitébutter gefüllt. Durch ihre Anwendung werden die Entzündungen der Scheidenchleimhaut kuriert und das Wurzelfassen der Bakterien verhindert, da sie ein mechanisches Hindernis bildet; außerdem wird der Beischlaf in jenen Frauen erleichtert, die an einer erregten Sekretion des Scheidenhumors leiden.

Durch die Anwendung eines die Karitébutter enthaltenden oder aus demselben Stoff bestehenden Gleitmittels für Präservative läßt sich ein Beischlaf sicher erreichen, der in den beiden Partner durch das Präservativ geschützt wird, da das Präservativ keine Entzündung verursacht, sondern den Beischlaf erleichtert; außerdem durch die Verwendung eines derartigen Präservativs werden die vorher bestehnden eventuellen Entzündungen der Scheidenchleimhaut gelindert Dieses Gleitmittel schützt die zarten Teile der Haut vor der Berührung mit dem Latex des Präservativs, damit läßt sich ein heilsames Ergebnis für den männlichen und weiblichen Geschlechtsapparate erreichen, das dem durch die Verwendung der oben genannten Gelatinekapseln erreichten Ergebnis ähnlich ist.

Das Gleitmittel sorgt für die Beschmierung der Außenwand oder auch der Innenwand des Präservativs.

Vorzugsweise ist das Gleitmittel an den beiden Wänden beschmiert, um seine heilsamen Ergebnisse sowohl an den männlichen als auch an den weiblichen Geschlechtsapparat zu übertragen.

Die Karitébutter ist viel ungesättiger als die Kakaobutter oder der Borneumtalg; sie ist aus den Samen von *Buthirospermum parkii* ausgezogen, deren dicke und an Fettstoffen reiche Kotyledonen auf kaltem Weg ausgepresst sind.

Ein zweiter Stoff, der als Gleitmittel für Präservative verwendbar ist, ist die Kakaobutter. Diese Butter enthält die Palmitinsäure,die Stearinsäure, die Oleinsäure, die Linoleinsäure und Glyzeride; sie ist aus den Samen von *Thembroma* cacao ausgezogen, die von den Regionen der Tropenamerika stammen. Die Ausziehung dieser Butter ist durch Auspressen auf warmem Weg der abgeschälten und gerösteten Samen ausgeführt; die sorgfältig ausgeführte Ausziehung mit Waschbenzin liefert ein weißes Erzeugnis.

Bei Raumtemperatur ist die Kakaobutter hart und zerbrechlich, ihre Farbe ist weiß und neigt zum Gelb, ihrer Geruch ist gefällig und ihrer Geschmack ist schmackhaft. Ihrer Säuregehalt ist niedriger als 2 Prozent.

Die Haupteigenschaft der Kakaobutter ist darin zu sehen, daß sie bei Raumtemperatur in festem Zustand bleibt, aber bei der Temperatur des menschlichen Körpers schmelzt sie; daraus folgt ihre verbreitete Anwendung in den Zäpfschen und in den erweichenden Mitteln für die Haut und die Lippen.

Ein zweiter Stoff, der als Gleitmittel für Präservative verwendbar ist, ist der Borneumtalg oder "grüne Butter". Dieser Stoff ist aus den Samen von *Shorea stenoptera* ausgezogen. Unter den Pflanzenbuttern ist diese die ähnlichste der Kakaobutter; daraus folgt ihre verbreitete Anwendung als Kakaobutter-Ersatz in den Lebensmitteln und in den Industrien der Kerzen und der Seifen.

Das Mowrafett (oder Illipébutter) ist aus der indischen Pflanze "*Bassia langifolia*" ausgezogen; wegen seines Sättigungsgrads und seiner Erstarrungstemperatur ist dieser Stoff der Karitébutter ähnlich; daraus folgt seine mögliche Anwendung als Gleitmittel für Präservative oder als Bestandteil einer Mischung von derartigen Gleitmitteln.

Der Pflanzentalg des China oder "Stillingiatalg" ist aus den Samen der Pflanze "*Stillingia sebifera*" ausgezogen; dieser Talg weist eine niedrige Säurezahl auf. Trotz des Mangels in diesem Talg der typischen Eigenschaften der Karitébutter, ist er als Gleitmittel für Präservative verwendbar.

Die aus der Pflanze "*Myristica officinalis* " ausgezogene Muskatnußbutter ist durch einen Gehalt (60-75%) an Miristiksäure gekennzeichnet, die oberflächenachtive Eigenschaften aufweist. Auch dieser Stoff ist als Gleitmittel für Präservative oder als Bestandteil eines derartigen Gleitmittels verwendbar.

Die vorliegende Tabelle legt die Mitteleigenschaften der oben genannten Pflanzenbutter dar.

| Analyse | Borneumtalg | Karitébutter | Mowrafett | Pflanzentalg des China | Muskatnußbutter |
|---|---|---|---|---|---|
| Spezifisches Gewicht bei 0 ° C. | 0,816 | 0,917 | 0,920 | 0,920 | 0,950 |
| Erstarrungstemperatur ° C | 22-30 | 17-27 | 18-25 | 25-35 | 41-44 |
| Schmelzpunkt ° C. | 28-37 | 23-32 | 23-31 | 43-46 | 43-48 |
| Erstarrungspunkt der Fettsäure ° C | 50-54 | 48-53 | 38-40 | 52-53 | 40-45 |
| Jodfarbzahl | 30-33 | 56-62 | 58-63 | 28-38 | 48-52 |
| Verseifungswert | 192-196 | 186-196 | 187-194 | 200-203 | 172-179 |
| Unverseifbarer Teil (Prozent) | <1 | 10 | 2-3 | variabile | 9-10 |
| Säuregehalt | 8-30 | 4-30 | 5-40 | 2-14 | 11-14 |

In der folgenden Tabelle sind die chemischen Zusammensetzungen verschiedener Pflanzenbutter dargelegt.

3

| Analyse | Kakaobutter | Borneumtalg | Karitébutter | Mowrafett |
|---|---|---|---|---|
| Jodfarbzahl | 38,7 | 33,2 | 59,1 | 63,9 |
| Fettsäure (Prozent in Gewicht) | | | | |
| Palmitinsäure | 24,4 | 18,0 | 5,7 | 23,7 |
| Stearinsäure | 35,4 | 43,3 | 41,0 | 19,3 |
| Erdnußsäure | - | 1,1 | - | - |
| Oleinsäure | 37,1 | 37,4 | 40,0 | 43,3 |
| Linoleinsäure | 2,1 | 0,2 | 4,3 | 13,7 |
| Glizeride (mol. %) | | | | |
| Palmitin-Stearine | 2 | 5 | 5 | 1 |
| Mono-ungesätti-ges-Palmitin-Stearine | 52 | 31 | - | - |
| Mono-ungesättige-Palmitine | 6 | 8 | - | 1 |
| Mono-ungesättige-Stearine | 19 | 40 | 34 | - |
| Di-ungesättige-Stearine | 12 | 13 | 45 | 30 |
| Di-ungesättige-Palmitine | 9 | 3 | 11 | 41 |
| Tri-ungesättige | - | - | 5 | - |

Durch eine Mischung von zwei oder mehreren Pflanzenbuttern wird ein Gleitmittel für Präservative erreicht; z. B. durch eine Mischung von Kakaobutter und Karitébutter.

Eine zum Zweck dosierte Mischung weist die besten Eigenschaften der verschiedenen Grundbestandteile auf.

Trotzdem ist es besser, daß die Mischung einen bedeutenden Hundertsatz an Karitébutter enthält, da diese Butter versuchmäßig bekannte Eigenschaften aufweist, durch die die Entzündungen der Scheidenchleimhaut gelindert werden; außerdem verhindert diese Butter das Wurzelfassen der Bakterien, da sie ein mechanisches Hindernis bildet, und erleichert den Beischlaf in jenen Frauen, die an einer erregten Sekretion des Scheidenhumors leiden.

Vorzugsweise enthält das Gleitmittel Bindemittel zur Besserung seiner Haftfestigkeit an den Außen-und Innnenwänden des Präservativs, wobei die Bindemittel die therapeutischen oder lindernden Eigenschaften des Gleitmittels nicht verändern; außerdem ist die Anwendung von mineralischen oder pflanzischen Pigmenten zur Lieferung einer gewünschten Farbe an das Gleitmittel vorgesehen. Ein der bevorzugten Bindemittel ist das Lezithin, das als Emulgiermittel wirkt.

Das Yoyobaöl ist aus den Samen von grossen Strauchen ausgezogen, die auf kaltem Weg ausgepresst sind. Diese Strauche wachsen in den trocken Regionen des Kaliforniens und waren als Yoyobabäume von den Indianern bekannt, die dieses Öls in großem Umfang zur Verhinderung der Dehydratation der den Winden und der Sonne der Wünstenländer ausgesetzten Haut anwandten.

Die Samen der Früchte sind an ungesättigen Fettsäuren reich, die viele Forscher als Vitamin F klassifizieren. Ihre vitaminreiche Beschaffenheit wird dadurch gezeichnet, daß von einer an ungesättigen Fettsäuren armen Diät ernährte Ratte Hautkrankheiten, Trockenheit und Faltigkeit der Haut aufweisen.

Daraus sind diese Samen in der menschlichen Pathologie zur Kur von vielen Hautkrankheiten (Ekzem, Akne, Dermatose) angewandt.

Das Yoyobaöl weist wirksame Eigenschaften für die Vernarbung und die Zeilenernährung der Haut auf; es wird nicht raffieniert angewandt; es ist unveränderlich und erstarrt bei niedrigen Temperatur.

Es ist als ein Wachs klassifiziert, da seine Fettsäuren durch einen Alkohol der Mono-Hydroxylgruppe esterifiziert werden.

Dieses Öl kann sowohl als ein pflanzisches Gleitmittel, als auch als ein Bestandteil eines aus einer Mischung von pflanzischen Buttern und/oder Ölen angewandt sein.

**Patentansprüche**

1. Gleitmittel für Präservative, **dadurch gekennzeichnet, daß** es aus einem oder mehreren fetten oder ölhatigen Pflanzenstoffen besteht.

2. Gleitmittel nach Anspruch 1, **dadurch gekennzeichnet, daß** einer der Pflanzenstoffe Karitene enthält.

3. Gleitmittel nach Anspruch 2, **dadurch gekennzeichnet, daß** einer der Pflanzenstoffe Karitébutter ist.

4. Gleitmittel nach Anspruch 1, **dadurch gekennzeichnet, daß** die Pflanzenstoffe Glizeride und Fettsäuren enthalten.

5. Gleitmittel nach Anspruch 1, **dadurch gekennzeichnet, daß** es wenigstens ein Bindemittel zur Besserung seiner Haftfestigkeit an den Außen-und Innnenwänden des Präservativs enthält, wobei die Bindemittel die therapeutischen oder lindernden Eigenschaften des Gleitmittels nicht verändern.

6. Gleitmittel nach Anspruch 5, **dadurch gekennzeichnet, daß** eines der Bindemittel das Lezithin ist, das als Emulgiermittel wirkt.

7. Gleitmittel nach Anspruch 5, **dadurch gekennzeichnet, daß** einer der Bestandteile Vitamine enthält.

8. Gleitmittel nach Anspruch 5, **dadurch gekennzeichnet, daß** einer der Bestandteile das Yoyobaöl ist.

9. Gleitmittel nach Anspruch 5, **dadurch gekennzeichnet, daß** es aus einer Mischung besteht, die wenigstens ein der folgenden Fette oder Öle enthält: Kakaobutter, Borneumtalg, Karitébutter, Mowrafett, Pflanzentalg des China, Muskatnußbutter.

10. Gleitmittel nach Anspruch 5, **dadurch gekennzeichnet, daß** die Mischung einen bedeutenden Hundertsatz an Karitébutter enthält.

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A | US-A-4 415 548 (K.PRAKASH REDDY)<br>* das ganze Dokument *<br>--- | 1,7-9 | A61K9/00<br>A61F6/04<br>A61K47/00 |
| A | BE-A-681 821 (TEMMLER-WERKE)<br>* das ganze Dokument *<br>--- | 1,7-9 | |
| A | US-A-4 981 686 (R.E.HARDY)<br>* das ganze Dokument *<br>--- | 1,7-9 | |
| A | US-A-4 410 517 (T.STILLMAN)<br>* das ganze Dokument *<br>--- | 1,7-9 | |
| A | EP-A-0 145 607 (L'OREAL)<br>* das ganze Dokument *<br><br>----- | 1-2,7-9 | |
| | | | **RECHERCHIERTE SACHGEBIETE (Int. Cl.5)**<br><br>A61K<br>A61F |

Der vorliegende Recherchenbericht wurde für alle Patentansprüche erstellt

| Recherchenort | Abschlußdatum der Recherche | Prüfer |
|---|---|---|
| DEN HAAG | 26 MAERZ 1993 | SCARPONI U. |